# FASCICULE DE BREVET EUROPEEN

(11) **EP 3 672 685 B1**
(45) Date de publication et mention de la délivrance du brevet: **26.08.2026**
(21) Numéro de dépôt: 18766299.4
(22) Date de dépôt: 18.09.2018
(51) Int. Cl.: A61N 5/02, A61N 5/04

(54) **MODULE D'ÉMISSION D'ONDES ÉLECTROMAGNÉTIQUES**
ELEKTROMAGNETISCHE WELLEN EMITTIERENDES MODUL
MODULE EMITTING ELECTROMAGNETIC WAVES

(30) Priorité: 18.09.2017 FR 1758634
(43) Date de publication de la demande: 01.07.2020
(73) Titulaire: Remedee Labs, 38240 Meylan (FR)
(72) Inventeur: SIBUE, Pierre-Yves, 38560 Jarrie (FR); FOERSTER, Michael, 38700 Corenc (FR); CROUZIER, David, 38240 Meylan (FR)
(74) Mandataire: LLR
(86) Numéro de dépôt international: PCT/EP2018/075232
(87) Numéro de publication internationale: WO 2019/053288

(56) Documents cités:
- EP-A1- 2 178 119
- WO-A1-2012/022538
- DE-A1- 102015 109 819
- KR-B1- 100 866 593
- US-A- 6 122 550
- US-A1- 2006 167 531
- US-A1- 2010 036 369
- US-A1- 2015 364 816
- HONGWEI LIANG ET AL: "Development of a 36 GHz millimeter-wave BGA package", MICROWAVE SYMPOSIUM DIGEST. 2000 IEEE MTT-S INTERNATIONAL BOSTON, MA, USA 11-16 JUNE 2000, PISCATAWAY, NJ, USA,IEEE, US, 11 June 2000 (2000-06-11), pages 65 - 68vol.1, XP032401791, ISBN: 978-0-7803-5687-0, DOI: 10.1109/MWSYM.2000.860886

## Description

L'invention concerne l'émission d'ondes électromagnétiques, notamment pour le traitement de la douleur.

En particulier, on s'intéresse aux dispositifs permettant d'émettre des ondes millimétriques, c'est-à-dire dans une bande de fréquence allant de 3 à 300 gigahertz.

Comprenant au moins un générateur d'ondes et une antenne permettant le rayonnement de ces ondes, ces dispositifs ont des applications variées, allant de l'intégration dans des systèmes complexes à des applications directes comme le traitement de la douleur, du stress ou des troubles du sommeil par l'émission d'ondes millimétriques vers la peau d'un patient.

On connaît un type de générateur d'ondes millimétriques comprenant un composant *« discret »* de type « *diode Gunn* ». Cependant, pour émettre des ondes d'une densité surfacique de plus de 5 mW/cm², ce type de produit est nécessairement volumineux, c'est-à-dire présentant un volume de l'ordre d'une dizaine de centimètres cubes. De plus, il présente un faible rendement énergétique et un fort échauffement.

On connaît également la technologie microélectronique « CMOS » (pour *« complementary metal-oxide-semiconductor* »), mais les générateurs d'ondes vendus actuellement et réalisés au moyen de cette technologie sont principalement réalisés en Silicon-germanium ou en Arséniure de Gallium et présentent un prix trop élevé pour un niveau de puissance trop faible, et sont vendus majoritairement sans antenne associée.

Concernant les antennes connues, elles sont conçues pour transmettre des ondes dans l'air et en champ lointain, et non en champ proche et vers la peau.

Les modules d'émission d'ondes actuels ne sont donc pas adaptés à certaines applications comme l'envoi d'ondes vers la peau d'un patient.

Les documents US2010/0036369.A1 et WO2012022538.A1 divulguent des modules d'émission d'ondes.

Un but de l'invention est donc de pallier aux inconvénients décrits ci-dessus.

À cet effet, on prévoit un module d'émission d'ondes selon la revendication 1
Ainsi, de petite taille, ce module peut être intégré dans un dispositif facilement manipulable, par exemple portatif tel qu'un smartphone ou une smartwatch, ou être intégré en grand nombre dans un dispositif plus complexe générant un rayonnement élevé sans prendre un espace important au sein du dispositif. De plus, à partir de 0.5 mW/cm², il est connu qu'un effet dans le traitement de la douleur est obtenu (voir la publication Rojavin MA, Radzievsky AA, Cowan A, Ziskin MC : *« Pain relief caused by millimeter waves in mice : results of cold water tail flick tests »*), de sorte qu'un seul de ces modules, de petite taille, peut permettre le traitement thérapeutique d'un patient ou servir à d'autres applications telles que la diminution du stress, la génération d'une sensation de bien-être ou la résolution de troubles du sommeil, sans prendre de la place, et avec un coût peu important.

Les ondes présentent une valeur de densité surfacique de puissance comprise entre 5 et 35 mW/cm².

Ainsi, les ondes émises respectent certaines normes limitant leur puissance en direction d'une peau humaine, mais la puissance reste suffisante pour qu'un effet, par exemple une diminution de la douleur ou une sensation de bien-être, soit obtenu.

La bande de fréquence de 3 à 120 gigahertz est particulièrement efficace pour le traitement de la douleur par ondes millimétriques. D'ailleurs, l'étude Radzievsky AA, Gordiienko OV, Alekseev S, Szabo I, Cowan A, Ziskin MC : « Electromagnetic millimeter waves for pain therapy. Evid Based Complement Alternat Med *»* tend à montrer que l'effet optimal d'un traitement par ondes millimétriques est obtenu avec une fréquence autour des 61,25 GHz et une densité surfacique de puissance d'environ 13 mW/cm².

Le module est apte à exposer aux ondes 2.5 cm² d'une surface, en particulier de peau, de manière continue. Alternativement, le module est apte à exposer aux ondes 2.5 cm² discontinus, c'est-à-dire plusieurs morceaux de surface répartis sur plusieurs endroits différents, qui tous ensemble, représentent 2.5 cm² de surface irradiée simultanément.

Par « exposer aux ondes », on veut également dire « irradier par les ondes ».

Par exemple, le module présente plusieurs antennes émettant des ondes simultanément, la zone de peau d'un patient couverte par l'ensemble des antennes et donc par le module représentant au moins 2.5 centimètres carrés continus, irradiés de façon homogène. Cela permet d'obtenir une surface irradiée en continu suffisante pour induire la réponse biologique attendue. Il est à noter que la fréquence émise par les différentes antennes n'est pas nécessairement la même. Les différentes antennes peuvent émettre des fréquences différentes, étant alimentées par différents ASICs. Les fréquences restent néanmoins dans la bande d'intérêt.

Avantageusement, il comprend une batterie rechargeable.

Ainsi, il fonctionne sans fil. Alternativement, il peut fonctionner de manière filaire, afin de délivrer des puissances plus élevées ou sur de plus longues durées.

Avantageusement, le module comprend un dissipateur thermique comportant au moins l'un des éléments suivants :
- un matériau à changement de phase ;
- du graphite ; et
- un matériau élastomère.

Ainsi, le dissipateur thermique permet de minimiser l'échauffement du module notamment s'il est intégré dans un dispositif appliqué sur la peau d'un patient. Cela permet là encore de respecter certaines normes mais aussi plus simplement d'éviter un échauffement trop important du module ou du dispositif dans lequel il pourrait être intégré.

De préférence, la surface étant une peau humaine ou animale, le module comprend un organe de détection de peau apte à signaler la présence ou l'absence de la peau à exposer aux les ondes, et de préférence apte à déterminer une distance séparant la peau et le module.

Ainsi, le module émet des ondes directement vers la peau du sujet seulement si la peau est détectée. Si la peau n'est pas détectée, ou si la distance est trop importante entre le module et la peau, aucune émission n'a lieu. De cette façon, on évite d'envoyer des ondes dans n'importe quelle direction et on économise de l'énergie. On peut également adapter la puissance ou d'autres paramètres des ondes émises en fonction de la distance estimée entre le module et la peau.

On prévoit également selon l'invention un dispositif portatif d'émission d'ondes électromagnétiques, comprenant un module décrit précédemment.

Ainsi, le dispositif peut être porté aisément par un patient humain ou animal et envoyer des ondes de manière prédéterminée ou sur commande, dans un but thérapeutique, en vue de générer une sensation de bien-être ou à toute autre fin. Le dispositif est d'autant plus aisé à être porté que le module d'émission est de faibles dimensions.

De préférence, le dispositif est apte à être porté au moins à l'un des endroits suivants :
- autour d'un poignet ;
- à une jambe ;
- à une cheville ;
- à un dos ;
- à une oreille ;
- dans une paume d'une main ; ou
- plus globalement tout endroit présentant une zone fortement innervée.

Ainsi, il est fixé à l'un de ces endroits, par exemple à la manière d'une montre autour du poignet, de manière à être porté sans inconvénient particulier par le patient. Concernant les zones fortement innervées, l'étude « Radzievsky AA, Rojavin MA, Cowan A, Alekseev SI, Ziskin MC. Hypoalgesic effect of millimeter waves in mice: Dependence on the site of exposure. Life sciences. 2000;66(21):2101-11 *»* a démontré l'effet thérapeutique avantageux de l'envoi d'ondes millimétriques dans de telles zones.

Nous allons maintenant présenter des modes de réalisation de l'invention à titre d'exemples non-limitatifs et en référence aux dessins dans lesquels :
- la figure 1 est un schéma global d'un mode de mise en oeuvre de l'invention ;
- les figures 2 et 3, 5 et 6 sont des illustrations d'un dispositif portatif selon un premier mode de réalisation de l'invention ;
- la figure 4 illustre un premier mode de mise en œuvre d'un tel dispositif ;
- les figures 7 à 15 sont des schémas des composants d'un module d'émission d'ondes selon un premier mode de réalisation ;
- les figures 16 et 17 sont des illustrations d'un tel module respectivement sans et avec dissipateur thermique ;
- la figure 18 est une illustration d'un rayonnement du module des figures 14 et 15 ;
- les figures 19 et 20 sont des illustrations d'utilisation selon des deuxième et troisième modes de mise en œuvre de l'invention ; et
- les figures 21 à 29 illustrent des composants d'un module selon d'autres modes de réalisation de l'invention.

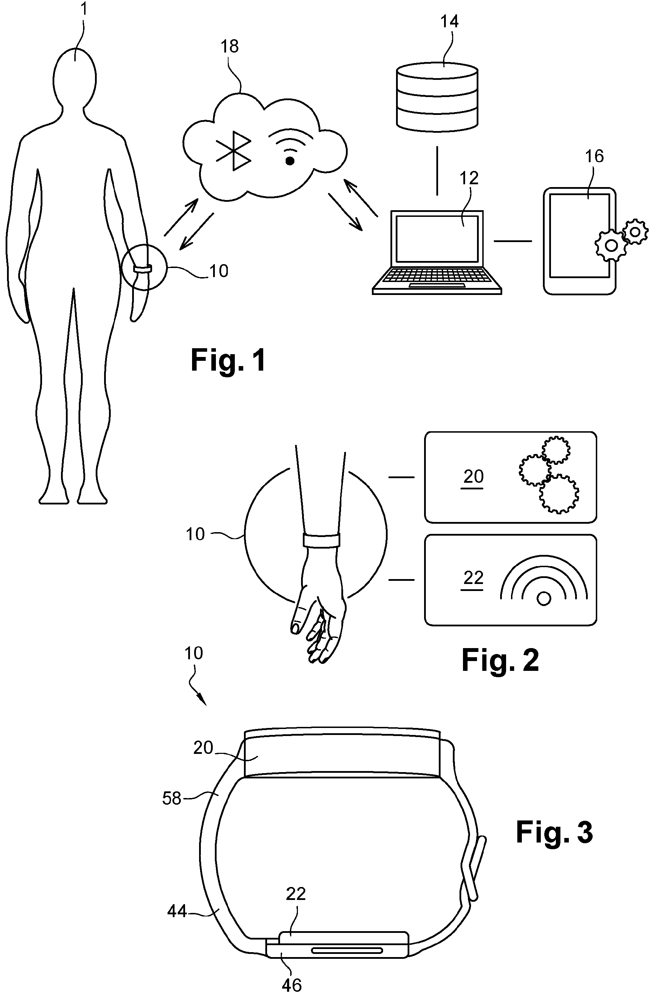

La figure 1 illustre le cadre général de l'invention. Le patient 1 est atteint de douleurs chroniques. Il porte un dispositif 10 selon un premier mode de réalisation et un premier mode de mise en œuvre de l'invention, qui traite ces douleurs en émettant des ondes électromagnétiques millimétriques vers la peau du patient 1, au niveau de son poignet. En l'espèce, ce dispositif 10 présente la forme générale d'une montre-bracelet, et est fixé autour du poignet de la même manière qu'une montre. Illustré à la figure 2 de façon schématique et aux figures 3, 5 et 6 plus en détails, le dispositif 10 comprend un module de commande 20 et un module d'émission d'ondes 22. Le dispositif 10 présentant la forme générale d'une montre, il peut s'agir d'une montre dans laquelle les modules 20 et 22 auraient été intégrés. A l'inverse, les fonctionnalités d'une montre peuvent être intégrées au dispositif 10.

Le module de commande 20 contrôle le module d'émission 22. Le module de commande 20 est activé par le patient, mais il peut aussi être programmé par le patient ou un autre utilisateur, sur le dispositif 10 directement par le bouton 23 ou via un terminal tel que l'ordinateur 12. Le bouton 23 est muni de diodes lumineuses qui peuvent être activées pour signifier un événement au patient, par exemple un manque de batterie ou le fonctionnement d'un programme particulier en cours. Le module de contrôle 20 est présent dans la partie supérieure du dispositif 10 tandis que le module millimétrique 22 d'émission d'ondes est situé dans la partie inférieure et a donc vocation à être en contact avec la peau inférieure du poignet.

On va maintenant décrire en détails le module d'émission d'ondes 22 intégré dans le dispositif 10. Il s'agit d'un module d'émission selon un premier mode de réalisation. Ce type de module, ainsi que ses autres modes de réalisation, peut être intégré dans tout type de dispositif visant à émettre des ondes, et pas uniquement dans le dispositif 10 en forme de montre-bracelet. Ses applications ne se limitent d'ailleurs pas au traitement de la douleur.

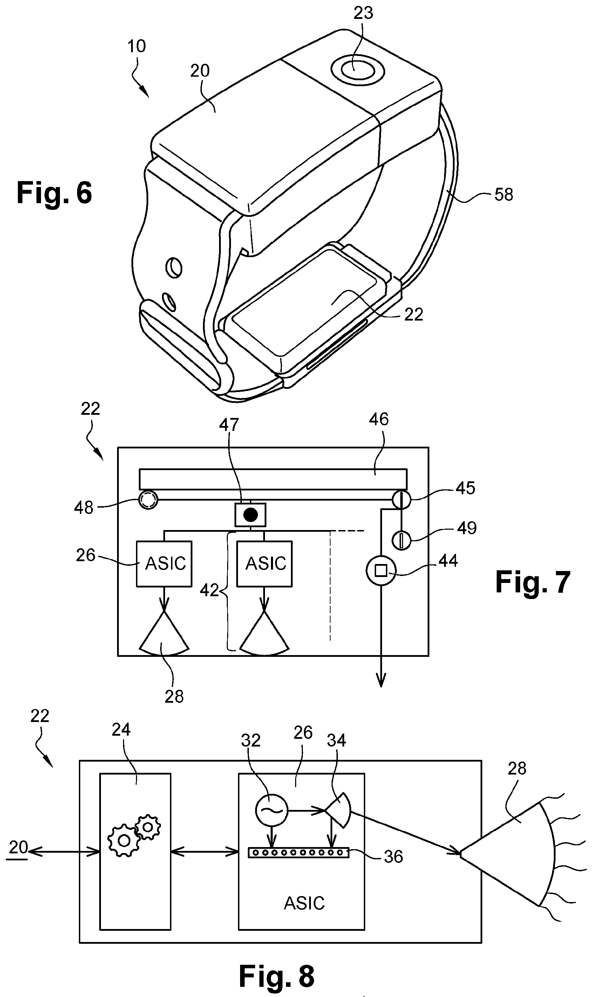

Ce module d'émission 22 illustré schématiquement à la figure 7 présente plusieurs couples circuit-antenne 42, un dissipateur thermique 46, un capteur de peau 44, une entrée d'alimentation 45, un organe de pilotage numérique 47, une horloge de référence 48, et un capteur de température 49.

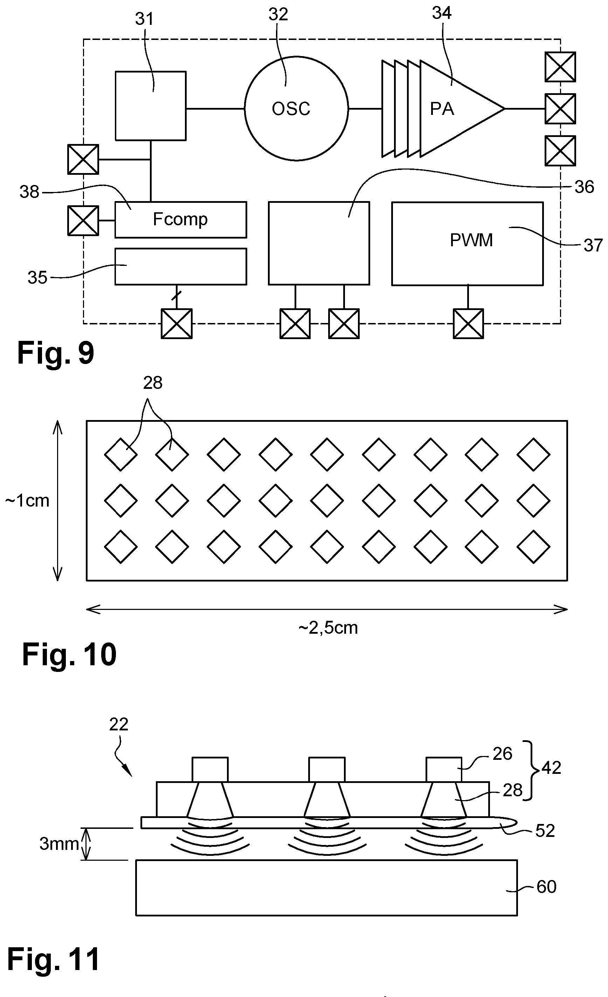

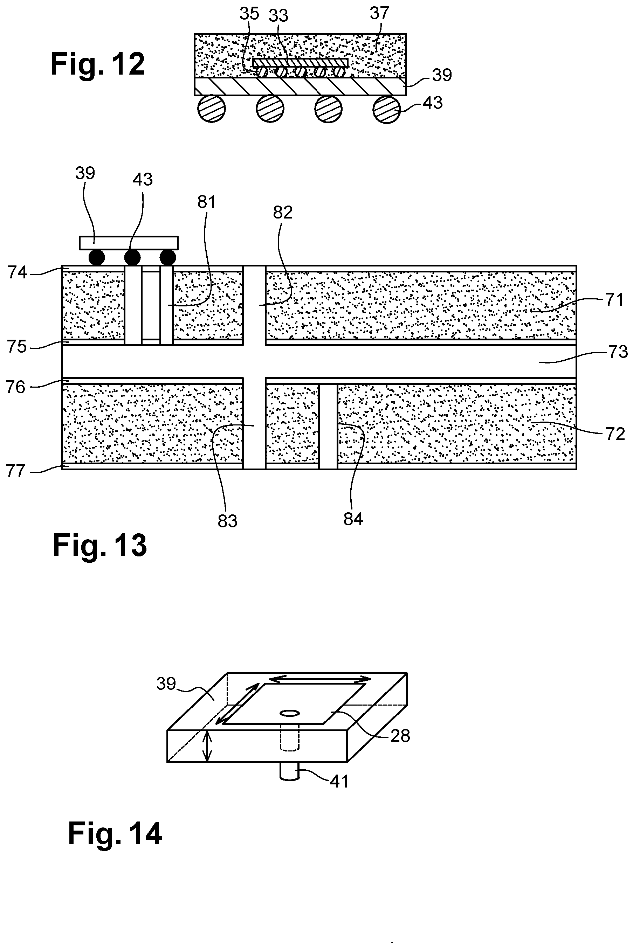

Chaque couple circuit-antenne 42, dont un est schématiquement illustré à la figure 8, présente une interface de contrôle 24 en liaison avec le module de contrôle 20, un ASIC (*« application-specific integrated circuit »,* ou circuit intégré pour application spécifique) 26 et une antenne 28. L'interface 24 peut être située au sein du module de contrôle 20. L'ASIC 26, tel qu'il est illustré à la figure 8, comprend un oscillateur 32, un amplificateur de puissance 34 et une partie numérique 36 de paramétrage et de contrôle du composant. Illustré plus en détail à la figure 9, il comprend également un diviseur de fréquence 31, , un bus de communication 35, un organe de pilotage en *« Pulse-width modulation »* (*PWM)* 37 et un comparateur de fréquence 38. L'oscillateur 32 permet de générer la fréquence de fonctionnement des ASIC. L'amplificateur permet d'amplifier ce signal afin que la puissance souhaitée soit disponible en sortie de composant. Cette puissance est réglable entre 0 et 20 mW. Sans difficulté, on peut concevoir qu'elle aille jusqu'à 60 mW. Le comparateur de fréquence et le diviseur permettent de vérifier la fréquence de fonctionnement. Le circuit de gestion des alimentations permet d'alimenter correctement l'ensemble des fonctions du composant. Enfin, l'organe de pilotage « PWM » permet d'émettre de façon continue ou discontinue le signal HF de sortie. L'ossature de l'ASIC est représentée à la figure 12. La fabrication de cet ASIC 26 est réalisée au moyen de la technologie « CMOS » (*« Complementary Metal Oxide Semiconductor »*), technologie connue de l'homme du métier et qui ne sera donc pas décrite en détails. Plus spécifiquement, les transistors sont de type *« CMOS 65 nanomètres* ». Alternativement, ils auraient pu être développés en silicium-germanium (SiGe) ou encore en arséniure de gallium (GaAs). En revanche, les technologies de type « diode gunn » ne permettent pas d'atteindre la taille minimale ainsi que le coût souhaités. L'ASIC 26 comprend ainsi un circuit intégré 33 en silicium dans un boitier 37 de type BGA (« *Ball Grid Array »*), un type de boitier bien connu de l'homme du métier, adapté sur mesure pour l'ASIC 26, le boitier comprenant également des billes 35 (dites *« bump* »).Comme illustré à la figure 13, le circuit 33 est soudé sur deux couches 71 et 72 de substrat « HF » 39 en PTFE (Polytétrafluoroéthylène) RO3003, par exemple de fabrication ROGERS, avec un agencement dit en « flip chip », ce qui permet de limiter au maximum les pertes de rayonnement électromagnétique à haute fréquence. Au lieu du RO3003, il pourrait s'agir de MT77 (par exemple de fabrication ISOLA) imprégnant des fibres de verre tissées, ou encore de RF301 (de fabrication TACONIC), ou de tout autre matériau ayant les même avantages techniques que ceux cités. Les deux couches 71 et 72 sont séparées par une couche 73 de RO4450F ainsi que par des couches de cuivre 74, 75, 76 et 77. En outre, des vias 81, 82, 83 et 84 effectuent les liaisons entre les différentes couches du substrat. Bien entendu, les types de couches et leur nombre pourraient être différents.

L'oscillateur 32 de fréquence est placé dans une cavité (non illustrée) au sein du boitier 37 ce qui permet de ne pas perturber la fréquence générée. La taille de ce boitier 37 BGA est en l'espèce de 2.2*2.2*0.9 millimètres. La connexion aux antennes 28 se fait au moyen de « *balls »* 43. Cet ensemble de composants permet de réduire au maximum les pertes d'ondes électromagnétiques. C'est l'antenne 28 qui émet des ondes électromagnétiques à destination de la peau du patient 1. L'agencement des ASIC, interface de contrôle et antennes au sein du module d'émission peut bien entendu être différent.

La connexion 41 terminale entre un ASIC 26 et son antenne 28 est visible à la figure 14. Une connexion coaxiale 41 assure ainsi la transmission de l'onde entre l'amplificateur de puissance 34 et l'antenne 28. Par antenne, on entend de manière générale toute forme d'élément rayonnant, à condition en l'espèce qu'il soit plan. On peut appeler ce type d'élément rayonnant un « patch ».

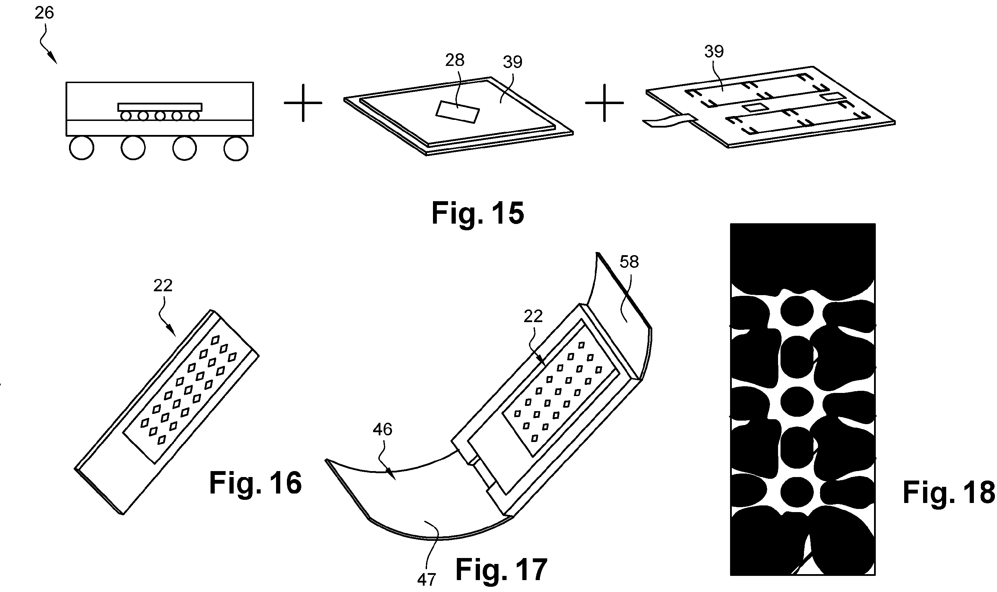

Comme le schématise la figure 15, l'ASIC 26 et l'antenne 28 sont disposés de part et d'autre du substrat 39.

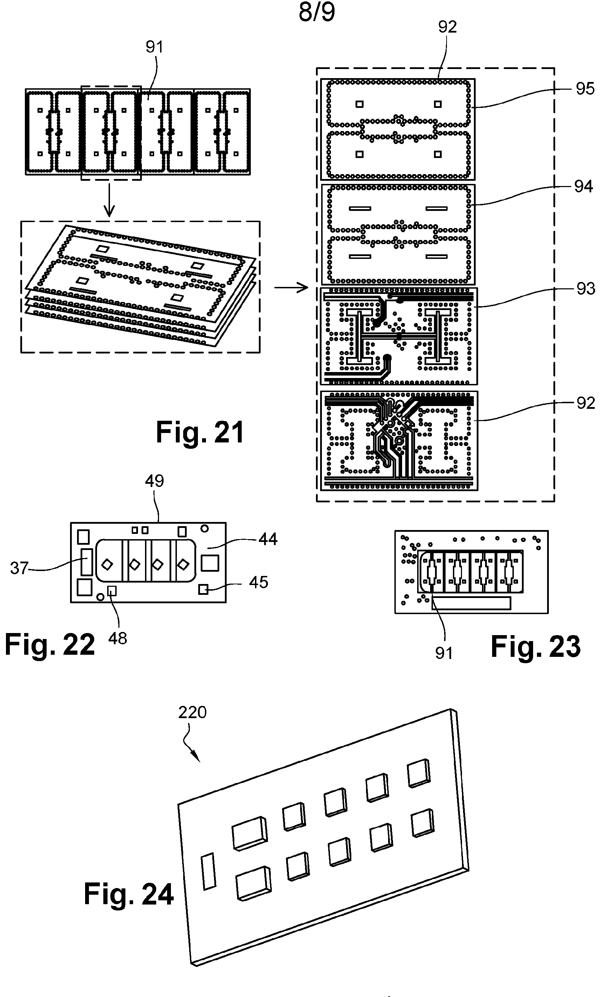

L'ensemble des antennes 28 forme un réseau d'antennes illustré à la figure 10. De forme rectangulaire ici, ce réseau d'antennes destiné à être disposé contre la peau du patient 1 ou à faible distance de celle-ci, mesure environ 2.5 centimètres de long pour environ 1 centimètre de large. Il est pourvu en l'espèce de 27 éléments rayonnants 28 fonctionnant en champ proche, à raison de trois rangées de neuf antennes, alignées verticalement et horizontalement les unes par rapport aux autres. Ces quantités et ces dispositions ne sont pas limitatives et d'autres peuvent être envisagées. Autour de ce réseau d'antennes, également appelé zone active, sont disposés les autres éléments de la figure 7 et de la figure 9, en particulier le capteur de température 49, le capteur de peau 44, l'horloge 48 et le module d'alimentation 45, comme illustré sur la figure 22 dans un mode de réalisation légèrement différent décrit plus bas. L'ensemble formé par ces éléments et la zone active située à l'intérieur mesure 37 *20 mm et forme le module d'émission 22, qui peut être intégré dans un dispositif tel qu'un bracelet.

Cet agencement permet à la zone active d'émettre des ondes de façon homogène sur 2.5 centimètres carrés de peau. Par *« homogène »,* on souhaite signifier que l'intensité des ondes arrivant sur la peau ne doit pas présenter un écart supérieur à environ 30% entre sa valeur maximale en un point et sa valeur minimale en un autre. On a représenté à la figure 18 le rayonnement sur la peau du patient émis par le dispositif dans un mode de fonctionnement normal. Les formes noires correspondent à un rayonnement compris entre 5 et 15 mW/cm², les formes blanches à un rayonnement inférieur à 5mW/cm². On observe que 75% de la surface est irradiée par des ondes de densité comprise entre 5 et 15 mW/cm². De manière générale, la densité de puissance peut être supérieure à 35 mW/cm², mais le dispositif est conçu de manière à ce que la gamme de puissance utilisée soit de l'ordre de 5 à 35mW/cm² en fonctionnement normal, notamment durant 30 minutes d'émission d'ondes en continu. Ce mode de fonctionnement est en effet le plus habituel, comme cela sera décrit plus bas.

La figure 11 illustre une application du module 22 d'émission d'ondes au niveau de la peau 60 du patient 1. Une distance de 3 millimètres sépare en espèce le module de la peau du patient. Bien que l'objectif soit d'apposer le dispositif sur la peau, il peut arriver qu'un léger espace se crée entre la peau et le dispositif. Par ailleurs, pour plus de confort et pour des raisons de biocompatibilité, une couche 52 de silicone sépare les antennes de la peau, de sorte que la peau n'a pas à supporter directement les antennes. Alternativement, il peut s'agir d'un autre matériau transparent aux ondes millimétriques tel que du polycarbonate. Cette couche 52 de silicone peut mesurer de 1 à 2 millimètres, la conception des antennes permettant que la couche 52 n'interfère que peu ou pas avec les ondes émises.

Au total, ce module d'émission d'ondes 22, qu'on peut appeler module millimétrique (les ondes étant dites *« millimétriques »* au vu de leur fréquence) ou carte millimétrique, mesure 37 millimètres de longueur, pour 20 millimètres de largeur et 3 millimètres d'épaisseur dans le présent mode de réalisation. Le volume du module millimétrique est donc de 2.96 centimètres cubes. Illustré à la figure 16, il est donc inférieur à quatre et même à trois centimètres cubes, ce qui permet de l'insérer dans des dispositifs de faible volume et légers, tel que le dispositif 10 en forme de montre-bracelet. Avec ce volume et l'agencement décrit présentant 27 antennes, les ASIC 26 développés, couplés aux antennes 28, permettent au module millimétrique d'émettre des ondes de fréquence située entre 3 et 300 gigahertz, de préférence entre 30 et 120 gigahertz. La fréquence préférée est de 61.25 GHz +/- 250 MHz. Dans tous les cas, la densité surfacique de puissance est d'au moins 0.5 milliwatts par centimètre carrés, et les ondes sont émises de façon simultanée sur une surface de peau de 2.5 centimètre carrés. Or, un traitement par ondes millimétriques est efficace à partir d'une densité de puissance de 0.5 milliwatts par centimètre carré, de préférence sur une surface d'au moins 1 centimètre carré. Le module décrit permet donc la réalisation du traitement en étant intégrable à tout dispositif et ce de manière facile au vu de son faible volume.

Il est entendu que les ASIC, les antennes, ainsi que l'ensemble du module millimétrique 22, peuvent présenter des volumes, des nombres différents et des agencements différents.

Ainsi, dans un second mode de réalisation illustré aux figures 21 à 23, les performances du module sont identiques. La différence est qu'un ASIC est couplé à quatre antennes sur une surface de 10*6.25 mm. Ce couple ASIC / antennes couvre donc une surface de peau de 0.625 cm², sur un substrat PCB de 1 mm d'épaisseur. Répété quatre fois côté à côte dans le module millimétrique illustré sur les figures 21 à 23, les quatre ASIC sont chacun placés dans un boitier « BGA » différent, dont la taille est de 2.2 mm x 2.2 mm x 1 mm. Le module, qui comprend alors deux rangées de huit antennes et 4 ASIC (4 boitiers), permet ainsi également d'irradier 2.5 cm² de surface de peau de manière continue.

Un réseau d'antennes 91 selon ce mode de réalisation est illustré à la figure 21. Dit « à cavités résonnantes », le réseau 91 comprend quatre couches. La couche 92 permet le routage des signaux numériques et d'alimentation. La deuxième couche 93 représente les lignes d'accès aux antennes. La troisième couche 94 représente les lignes de couplage. Enfin, la quatrième couche 95 est celle d'où seront émises les ondes. Ce réseau d'antennes est également mis en œuvre dans le mode précédent, la seule différence étant le nombre d'antennes et d'ASIC, et donc l'agencement de ces éléments.

Alternativement, en plaçant les couples ASIC / quatre antennes de manière séparés à différents endroits de la peau du patient, cette surface de 2.5 cm² est irradiée, mais en plusieurs parties distinctes. De même, chacun de ces couples peut être utilisé indépendamment, afin d'assurer un confort d'utilisation plus grand ou de s'intégrer dans des applications qui nécessitent une surface plus petite, ou une puissance plus faible.

Le capteur de peau 44 des modes de réalisations décrits utilise une mesure de type capacitive permettant de déterminer que la peau du patient est positionnée à proximité du module millimétrique 22. Sa structure est connue de l'homme du métier et n'est pas limitée à une mesure capacitive, tout capteur de peau miniaturisable étant admissible. Connecté à l'interface de contrôle 24 et/ou au module de contrôle 20, le capteur de peau 44 détermine la présence ou l'absence de peau humaine ou animale. Il est également apte à déterminer la distance séparant la peau et le module millimétrique. A 3 millimètres ou moins, l'émission d'onde est permise. Sinon, le module de contrôle 20 peut empêcher l'envoi d'ondes. L'objectif ici est d'empêcher l'envoi d'ondes de manière inefficace, afin d'une part de maîtriser la direction des ondes émises, et d'autre part de faire des économies d'énergie. Dans le premier mode de réalisation, le capteur de peau 44 est situé en dehors du module, sur un flanc du dispositif 10.

Le module millimétrique 22 peut comprendre en outre une batterie rechargeable. De préférence, l'ensemble du dispositif comprenant le module 22, tel que le dispositif 10, présente une batterie alimentant à la fois le module de contrôle 20 et le module d'émission d'ondes 22. Cette batterie peut être rechargée classiquement sur secteur ou de toute autre manière. Il est bien entendu intéressant que son autonomie soit de plusieurs heures, voire de plusieurs jours, afin que le dispositif portatif du patient visant à traiter sa douleur soit plus commode à utiliser.

Bien entendu, certains des composants du module peuvent être placés à l'extérieur de celui-ci pour mieux interagir avec le dispositif comprenant le module, comme la batterie.

En dehors du module de contrôle 20, du module millimétrique 22 et du capteur de peau 44, le dispositif 10 présente d'autres composants décrits maintenant.

La nappe 58 de la figure 3 est souple et vise à s'adapter à la forme et à la taille du poignet comme le ferait un bracelet classique de montre.

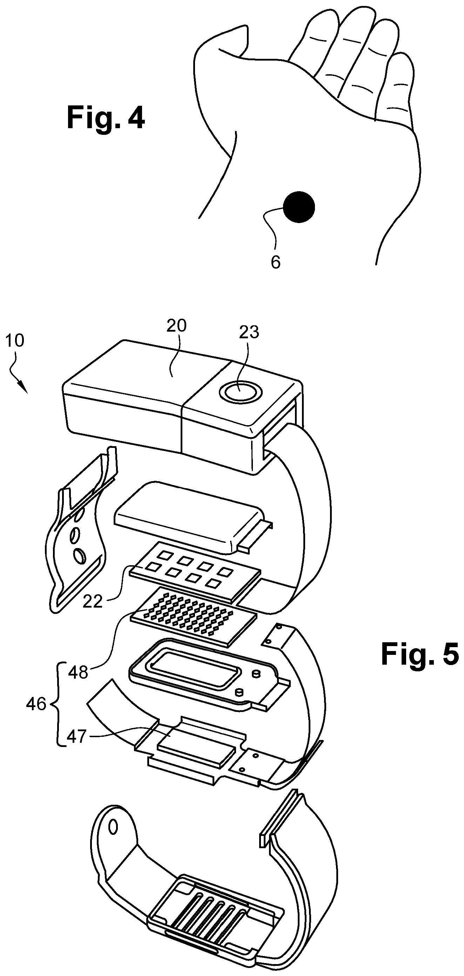

Le dispositif 10 comprend également un dissipateur 46 illustré à la figure 5, qui peut être considéré comme faisant partie du module millimétrique 22. En l'espèce, il se trouve à l'extérieur de ce module, et comprend une nappe souple 47 et un tampon thermique 48, les deux composants s'insérant au sein du bracelet du dispositif 10. A la nappe 47 est associée du graphite et du caoutchouc. Le caoutchouc permet à la nappe d'être souple et donc adaptable au bracelet. Le graphite est léger et présente une bonne conductivité thermique. La nappe 47 peut être constituée d'un autre matériau élastomère que le caoutchouc. Elle peut d'ailleurs présenter un tout autre matériau, son intérêt ici étant qu'il soit souple pour s'adapter à la forme du dispositif. Le tampon 48 comprend un matériau à changement de phase. Ainsi, lors du dégagement de chaleur dû au fonctionnement du dispositif, le matériau à changement de phase absorbe une partie des calories évacuées et permet le maintien global de la température. Le dissipateur est agencé avec le dispositif dans le but de maintenir en dessous de 43°C la température de la zone du corps environnante pour un fonctionnement du dispositif d'environ 30 minutes en continu. Cette température de 43°C correspond à des normes de températures maximales fixées par certaines autorités, et c'est pourquoi l'agencement du dispositif est conçu afin de la respecter. Il pourrait ainsi être conçu différemment si la température maximale autorisée était plus élevée . La température est surveillée grâce au capteur de température du module millimétrique 22.

Le dispositif 10 comprend également un organe (non représenté) de détermination d'impédance de la peau. Cet organe peut faire partie du module millimétrique 22.

Concernant la fréquence des ondes émises par le dispositif 10 grâce au module 22, elle peut être située entre 3 et 300 gigaherz pour un traitement efficace. Toutefois, la fréquence du dispositif décrit varie de préférence entre 30 et 120 gigaherz, avec une fréquence préférentielle située aux alentours de 60 gigaherz, en particulier vers 61.25 gigaherz.

Pour chaque composant, ses propriétés diélectriques, telles que sa permittivité, sa conductivité, sa tangente de perte, ont dû être prises en compte pour la conception du module 22 et du dispositif 10. Des simulations et essais hors de la plage de fonctionnement nominale des transistors des ASIC de type CMOS 65nm ont été effectués, et ne remettent pas en cause la durée de vie des composants au regard de la mise en œuvre du traitement par ondes millimétriques qui va être décrite ci-après.

On va maintenant s'intéresser à la mise en œuvre du traitement de la douleur chez le patient.

Ce traitement vise à émettre des ondes au niveau d'une zone de peau du patient. L'émission dure généralement 30 minutes, à raison d'une émission à deux par jour. La fréquence, de préférence entre 30 et 120 gigahertz, est prédéterminée. Elle peut éventuellement varier au cours d'une émission, de même que la densité de puissance surfacique qui évolue généralement entre 5 et 35mW/cm², mais peut être inférieure ou supérieure à cette gamme. Bien évidemment, tout autre type de traitement est envisageable, notamment avec des émissions plus longues et/ou plus fréquentes.

Dans un premier mode de mise en œuvre, les ondes sont émises par le module 22 intégré dans le dispositif 10 en forme de montre bracelet au niveau du poignet, zone fortement innervé et peut se placer sur le « point péricardium six » référencé 6 sur la figure 4, qui est un point d'acupuncture connu. Il a en effet été démontré que l'émission d'ondes vers des points d'acupuncture était particulièrement efficace quant au traitement de la douleur. De plus, de très bons résultats sont également atteints pour les zones particulièrement innervées. En effet, la stimulation des terminaisons nerveuses situées sous la peau induit un ensemble d'actions physiologiques appelé « réponse systémique », actions qui induisent à leur tour la synthèse d'opioïdes endogènes (dont l'enképhaline) elles-mêmes responsable de la diminution de la douleur. Ainsi, plus l'émission d'ondes a lieu dans une zone présentant une forte densité de terminaisons nerveuses, plus le traitement a de chances d'être efficace. Le point pericardium six est un point d'acupuncture en même temps situé dans une zone riche en terminaisons nerveuse. L'intérêt d'un dispositif émettant des ondes à ce niveau est donc maximal.

Par ailleurs d'autres bénéfices potentiels décrits dans la littérature associé à cette augmentation de la synthèse d'opioïdes sont connus, tel que la diminution de la fréquence cardiaque, du stress, une amélioration du sommeil, ou encore un effet euphorisant. De tels bénéfices peuvent donc être tirés du dispositif 10.

La fréquence, la durée, et la puissance des ondes est paramétrable au moyen du module 20 du dispositif 10. Comme l'illustre la figure 1, il peut être programmé à l'avance au moyen d'un terminal, par exemple un ordinateur 12, qui peut communiquer avec lui au moyen de tout réseau de télécommunication, tel qu'un lien de type Bluetooth ou Wifi 18. L'ordinateur 12 comprend une base de données 14 sur laquelle est enregistré un programme 16 mettant en œuvre le ou les procédés ayant un lien avec l'invention, ainsi que diverses données permettant la mise en œuvre de l'invention, notamment des données entrées par le patient 1 et des données obtenues par le dispositif 10.

Par ailleurs, en déterminant l'impédance de la peau grâce à l'organe de détection d'impédance, ce dernier transfère au module de contrôle 20 une donnée caractéristique de la peau du patient. Des paramètres des ondes émises par le module 22 peuvent alors être modifiés automatiquement via l'organe de contrôle 20, grâce au programme 16, ou manuellement par le patient ou un autre utilisateur. Ainsi, le dispositif 10 s'adapte à la peau du patient. Autrement dit, le champ électromagnétique crée est asservi aux caractéristiques de la peau. Il peut par ailleurs être modifié également en fonction de la distance mesurée entre la peau et le dispositif, via le détecteur de peau 44. Le dispositif peut comprendre d'autres organes déterminant et traitant d'autres données obtenues directement sur le patient, qui peuvent servir à adapter les paramètres des ondes émises telles que la puissance, la fréquence et la durée d'émission.

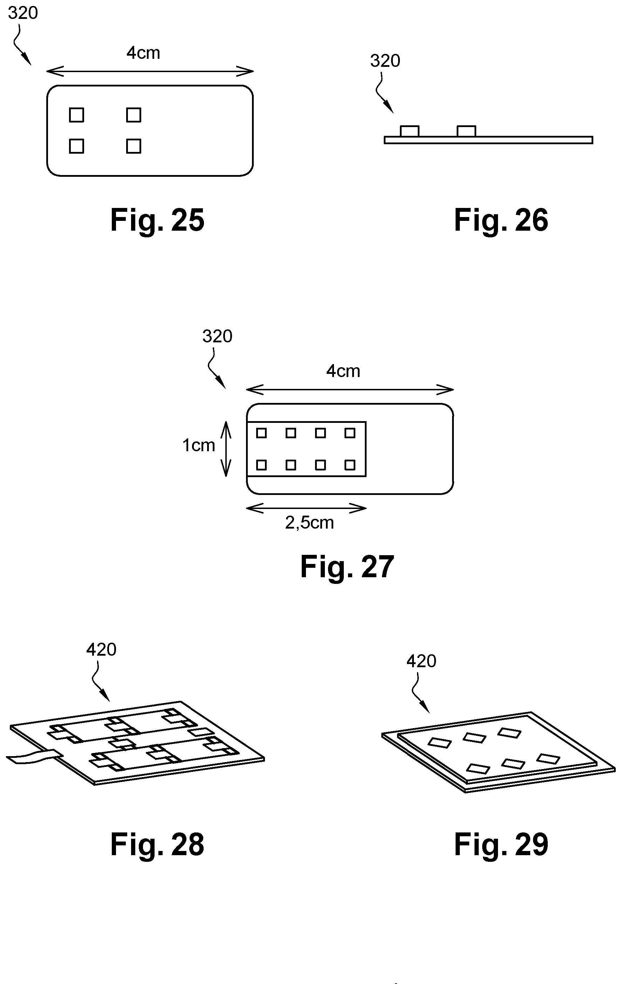

D'autres modes de réalisations du module d'émission sont illustrés aux figures 24 à 29. Ils se différencient des modes précédents par leur nombre d'ASIC et d'antennes. Le module de la figure 24 présente ainsi 8 ASIC. De plus, à un ASIC peut correspondre un ou plusieurs éléments rayonnants. Ainsi, le module 320 présente 4 ASIC pour 8 éléments rayonnants, à raison de 2 éléments rayonnant pour 1 ASIC. Enfin, le module 420 présente 6 ASIC et 6 éléments rayonnants.

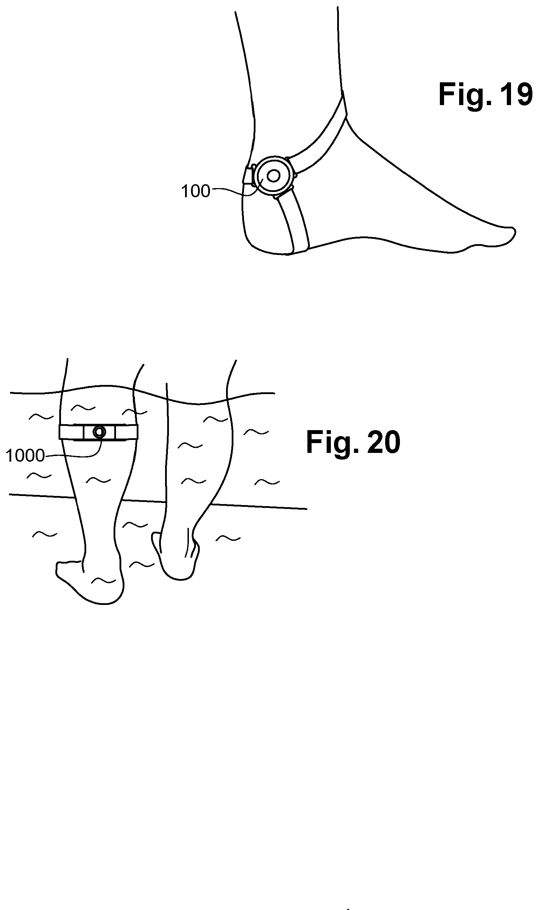

Par ailleurs, le module d'émission peut aussi être intégré dans un autre dispositif, par exemple destiné à être porté par le patient dans une autre partie du corps. La figure 19 illustre ainsi un dispositif 100 selon un deuxième mode de mise en œuvre comprenant les modules de contrôle et d'émission placé sur la cheville, tandis que la figure 20 illustre un tel dispositif 1000 selon un troisième mode de mise en oeuvre placé au niveau du mollet. Dans ces deuxième et troisième modes de mise en œuvre, les ondes sont donc émises au niveau d'autres zones du corps du patient au moyen de dispositifs qui diffèrent du dispositif 10 essentiellement pour s'adapter à la zone de peau visée. Dans tous les cas, la miniaturisation des modules permet au dispositif d'être léger et peu volumineux, de sorte qu'il est facile à porter et peu contraignant.

Au sein de ce module d'émission, des modifications sont possibles. Par exemple, la structure du réseau d'antennes peut être différente et présenter une ligne d'alimentation de type « micro ruban » ou une sonde coaxiale. Les antennes pourraient être à « longs slots ».

Le module de commande peut également être intégré au module d'émission électromagnétique.

On a donc présenté plusieurs modes de réalisation et de mise en œuvre, qui permettent tous d'émettre des ondes électromagnétiques présentant une densité surfacique de puissance d'au moins 0.5 milliwatts par centimètre carré de surface, une valeur de fréquence comprise entre 3 et 120 gigahertz, et sur simultanément au moins 2.5 centimètres carrés d'une surface, que ce soit en continu ou sur plusieurs morceaux de surface distincts.

Indépendamment de tout traitement de la douleur, le module d'émission d'ondes, éventuellement avec le module de contrôle, peut être intéressant pour l'envoi d'ondes dans d'autres finalités, par exemple en vue d'améliorer le sommeil, puisqu'il est particulièrement miniaturisé, et donc léger. Il peut donc être intégré à n'importe quel dispositif où l'envoi d'ondes millimétriques vers une surface ou dans une direction quelconque est nécessaire.

Par ailleurs, le module d'émission, ou le module de contrôle, et/ou le dispositif intégrant ces modules, peut être contrôlé à distance, à partir d'un terminal tel qu'un ordinateur, mais aussi à partir d'un terminal mobile. Par exemple, une application mobile comprenant un programme de traitement de la douleur peut être enregistrée sur le terminal mobile, de manière à ce que le patient programme lui-même son traitement, par exemple la puissance, la fréquence, la durée et le moment d'envoi des ondes, ou que son médecin ou tout auxiliaire médical programme à distance ces paramètres. Dans ce cas, le terminal comprend un logiciel présentant une ou des interfaces permettant à l'utilisateur du terminal de paramétrer le dispositif. Le programme permettant la mise en œuvre de l'invention peut être téléchargé via un réseau de télécommunication.

On peut ajouter que le module d'émission ainsi que le dispositif le comprenant peuvent aussi être utilisés en vue de diminuer le stress du patient ou même apporter une sensation de bien-être.

Corollairement, on peut prévoir l'utilisation de l'émission d'ondes électromagnétiques dans le cadre d'un programme d'amélioration du problème à résoudre perçu par le patient. Le programme peut consister en l'engagement sur une série d'utilisations encadrées du traitement avec évolution des paramètres d'exposition (fréquence, puissance...). On peut par exemple imaginer une séance découverte, suivie d'une séance adaptée au ressenti des personnes et de la puissance de l'effet perçu. On pourrait aussi adapter les séances suivantes en fonction de l'effet mesuré si des capteurs permettent de mesurer cet effet. Enfin la séance de traitement pourrait être déclenchée de manière programmée par l'utilisateur, ou de manière automatique si des capteurs permettent d'en mesurer le besoin.

Bien entendu, on pourra apporter à l'invention de nombreuses modifications sans sortir du cadre de celle-ci.

## Revendications

1. Module d'émission d'ondes électromagnétiques (22 ; 220 ; 320 ; 420) comprenant au moins un circuit intégré (26) apte à générer des ondes électromagnétiques, le circuit comportant un amplificateur de puissance (34), le module comprenant également un dissipateur thermique (46) comportant une nappe souple (47) et un tampon thermique (48), dans lequel le circuit (26) est intégré dans un boitier (37) de type BGA et comprend un oscillateur de fréquence (32), le module présentant un volume total inférieur à 3 centimètres cubes et étant apte, lorsqu'il est disposé au niveau d'une surface (60), à émettre les ondes électromagnétiques, les ondes présentant une densité surfacique de puissance comprise entre 5 et 35 mW/cm² de surface et présentant une valeur de fréquence située entre 61 et 61.5 gigahertz, le module étant apte à exposer simultanément aux ondes au moins 2.5 centimètres carrés de la surface.

2. Module (22 ; 220 ; 320 ; 420) selon l'une quelconque des revendications précédentes, comprenant une batterie rechargeable.

3. Module (22 ; 220 ; 320 ; 420) selon l'une quelconque des revendications précédentes, dans lequel le dissipateur thermique (46) comporte au moins l'un des éléments suivants :
- un matériau à changement de phase ;
- du graphite ; et
- un matériau élastomère.

4. Module (22 ; 220 ; 320 ; 420) selon l'une quelconque des revendications précédentes, dans lequel la surface (60) étant une peau humaine ou animale, le module comprend un organe de détection (44) de peau humaine ou animale, le module étant apte à signaler la présence ou l'absence de la peau à exposer aux ondes, et de préférence apte à déterminer une distance séparant la peau (60) et le module (10 ; 100 ; 1000).

5. Dispositif portatif (10 ; 100 ; 1000) d'émission d'ondes électromagnétiques, **caractérisé en ce qu'**il comprend un module selon l'une quelconque des revendications précédentes.

6. Dispositif (10 ; 100 ; 1000) selon la revendication précédente, apte à être porté au moins à l'un des endroits suivants :
- autour d'un poignet ;
- à une jambe ;
- à une cheville ;
- à un dos ;
- à une oreille ; ou
- dans une paume d'une main.

## Patentansprüche

1. Modul (22; 220; 320; 420) zum Emittieren elektromagnetischer Wellen, umfassend mindestens einen integrierten Schaltkreis (26), der imstande ist, elektromagnetische Wellen zu generieren, wobei der Schaltkreis einen Leistungsverstärker (34) umfasst, wobei das Modul ferner einen Wärmeableiter (46) mit einer flexiblen Schicht (47) und einem Wärmepuffer (48) umfasst, wobei
der Schaltkreis (26) in ein Gehäuse (37) des BGA-Typs integriert ist und einen Frequenz-Oszillator (32) umfasst, wobei das Modul ein Gesamtvolumen kleiner als 3 Kubikzentimeter aufweist und, wenn es im Bereich einer Oberfläche (60) angeordnet ist, zum Emittieren der elektromagnetischen Wellen imstande ist, wobei die Wellen eine flächenbezogene Leistungsdichte zwischen 5 und 35 mW/cm² aufweisen und einen Frequenzwert aufweisen, der zwischen 61 und 61,5 Gigahertz liegt, wobei das Modul imstande ist, mindestens 2,5 Quadratzentimeter der Oberfläche gleichzeitig gegenüber den Wellen zu exponieren.

2. Modul (22; 220; 320; 420) nach einem der vorhergehenden Ansprüche, umfassend eine wiederaufladbare Batterie.

3. Modul (22; 220; 320; 420) nach einem der vorhergehenden Ansprüche, wobei der Wärmeableiter (46) mindestens eines der folgenden Elemente aufweist:
- ein Phasenwechselmaterial;
- Graphit; und
- ein Elastomermaterial.

4. Modul (22; 220; 320; 420) nach einem der vorhergehenden Ansprüche, wobei, wenn die Oberfläche (60) eine menschliche oder tierische Haut ist, das Modul ein Erkennungsmittel (44) zum Erkennen menschlicher oder tierischer Haut ist, wobei das Modul imstande ist, das Vorhandensein oder Nichtvorhandensein der gegenüber den Wellen zu exponierenden Haut anzuzeigen, und vorzugsweise imstande ist, eine Distanz zwischen der Haut (60) und dem Modul (10; 100; 1000) zu ermitteln.

5. Tragbare Vorrichtung (10; 100; 1000) zum Emittieren elektromagnetischer Wellen, **dadurch gekennzeichnet, dass** es ein Modul nach einem der vorhergehenden Ansprüche umfasst.

6. Vorrichtung (10; 100; 10) nach dem vorhergehenden Anspruch, das an mindestens einer der folgenden Stellen tragbar ist:
- um ein Handgelenk;
- an einem Bein;
- an einem Knöchel;
- an einem Rücken;
- an einem Ohr; oder
- in einem Ballen einer Hand.

## Claims

1. Electromagnetic wave emission module (22; 220; 320; 420) comprising at least one integrated circuit (26) capable of generating electromagnetic waves, the circuit comprising a power amplifier (34), the module also comprising a heat sink (46) comprising a flexible sheet (47) and a thermal pad (48), wherein
the circuit (26) is integrated in a BGA-type package (37) and comprises a frequency oscillator (32), the module having a total volume of less than 3 cubic centimeters and being capable, when positioned at a surface (60), of emitting the electromagnetic waves, the waves having a surface power density of between 5 and 35 mW/cm² of surface and having a frequency value of between 61 and 61.5 gigahertz, the module being capable of simultaneously exposing at least 2.5 square centimeters of the surface to the waves.

2. Module (22; 220; 320; 420) according to any one of the preceding claims, comprising a rechargeable battery.

3. Module (22; 220; 320; 420) according to any one of the preceding claims, wherein the heat sink (46) comprises at least one of the following elements:
- a phase-change material;
- graphite; and
- an elastomeric material.

4. Module (22; 220; 320; 420) according to any one of the preceding claims, in which the surface (60) being a human or animal skin, the module comprises a human or animal skin detection member (44), the module being capable of signaling the presence or absence of the skin to be exposed to the waves, and preferably capable of determining a distance separating the skin (60) and the module (10; 100; 1000).

5. Portable device (10; 100; 1000) for emitting electromagnetic waves, **characterised in that** it comprises a module according to any one of the preceding claims.

6. Device (10; 100; 1000) according to the preceding claim, suitable for being worn at least at one of the following locations:
- around a wrist;
- to a leg;
- to an ankle;
- to a back;
- on an ear; or
- in a palm of a hand.
